# EUROPEAN PATENT APPLICATION

(11) **EP 1 770 551 A1**
(43) Date of publication of application: **04.04.2007**
(21) Application number: 05754673.1
(22) Date of filing: 08.06.2005
(51) Int. Cl.: G06F 17/30, A61B 17/00

(54) **TASK PLANNING SYSTEM AND METHOD FOR USE IN COGNITIVE ABILITY-RELATED TREATMENT**

(30) Priority: 10.06.2004 ES 200401420
(71) Applicant: Educamigos, S.L., 08021 Barcelona (ES)
(72) Inventor: LLORENTE PEREZ, Juan Ramon, E-08021 Barcelona (ES)
(74) Representative: Barroso Sánchez-Lafuente, Ignacio M.
(86) International application number: PCT/ES2005/000326
(87) International publication number: WO 2005/124589

(57) **Abstract**

The invention relates to a task planning system and method for use in cognitive ability-related treatment. The inventive system comprises: at least one central control and processing unit (B1) which manages a database of users and definitions of exercises and methods for cognitive ability-related treatment; at least one client computer (A1) for the user performing exercises; and communication means between the central control and processing unit (B1) and the user client computer, which are designed to (i) send exercises from the central control and processing unit (B1) to the user client computer (A1) that are proposed by decision-making means and (ii) send the results of the exercises from the user client computer (A1) to the control and processing unit (B1). The aforementioned decision-making means comprise an expert system (B2) which is implemented in the central control and processing unit (B1) and which is designed to determinate automatically the exercises to be performed by the user in a personalised manner, such that they are adapted to the user's development.

## Description

### Technical field of the invention

The present invention relates to an interactive and multimedia method and system especially designed for task planning in cognitive ability-related treatment, the stimulation, development and rehabilitation of people's various cognitive abilities. In particular, the present invention relates to a task planning system for use in cognitive ability-related treatment, of the type which comprises at least one central control and processing unit (B1) which manages a database of users and definitions of exercises and methods for cognitive ability-related treatments.

The main objective is to offer a practical, economic solution without space-time barriers, both to professionals and specialists in the treatment of cognitive deficiencies, as well as the family members and carers of the persons who suffer from these lacks.

This system is based on the use, storage, management and communication of interactive activities designed to be able to treat a wide type of cognitive deficiencies, whether congenital or acquired.

### Background of the invention

These days new technologies accompany us in many of our activities, those of the home, those of the office and also leisure and health activities.

In just a few years, on-line courses have transformed education and e-learning is increasingly becoming more important. Most on-line courses are focused on the distribution of information through varied documentation by tutorials, step by step wizards, quick guides... in the format of hypertext (HTML). Interaction with the course tutors/teachers is usually made via e-mail and in some cases by telephone.

Due to the digital revolution and, in consequence, the greater adoption and adaptation of e-learning as another learning media, the potential for the systems called agents is increasing.

Agents are defined as software entities which interact autonomously, providing greater interactivity with the user and, consequently, more personalised and effective attention is received.

Patent US005711671 A relates to *"Automated cognitive rehabilitation system and method for treating brain injured patients".* Said patent discloses a method and a system according to the introduction of claim 1, to communicate patients with specialists, so that it is the professional that decides which exercises the patient should perform, avoiding necessary trips.

According to the method, a client device is connected to a remote computer via a telephone modem. Software is executed locally in the client device and receives the exercises pre-established by the specialist one by one.

As is gathered from the specification of said patent, the results of the exercises are received by the specialist by fax or modem, it being possible to store the data for later research but without performing further operations, they are stored only to see the patient's development.

The system described cannot propose to patients a work plan based on the combination of several exercises with different use parameters, nor undergo automatic modifications by any of the parties. It is the specialist who at all times decides what exercises the patient should do unitarily. This is an important drawback since it does not take advantage of the potentiality of the data obtained and hinders the achievement and maintenance of the optimum point of cognitive stress of the patient, which encourages increasing and continuous learning without producing frustration due to difficulty or boredom due to simplicity.

On the other hand, as said patent claims, the agent user interface is stored locally, both in the specialist's and the patient's personal computer, which makes it necessary for the program (software) to be installed on each computer and means that the devices should store information on the interface formats. Therefore, said devices should implement a protocol for the transmission of said interfaces. This means that modifications of the exercises are costly, not automatic, and involve less personalisation in the treatment.

### Explanation of the invention

The present invention resolves all the aforementioned drawbacks, also providing new technical characteristics which are described below.

According to a first aspect of the invention, a task planning system is provided for use in the treatment of cognitive abilities which may have been lost or deteriorated due to congenital or acquired causes and that, essentially, it is characterized in that said decision-making means comprise an expert system which is implemented in the central control and processing unit, designed to determine automatically the exercises to be performed by the user in a personalised manner, such that they are adapted to his/her development and to modify the information content of the central control and processing unit memory.

In accordance with another characteristic of the system of the invention, said database contains elementary exercises, from which the expert system determines an exercise table.

Said exercise tables are preferably combinations of elementary exercises and criteria of parametrization, such as the time of duration thereof, the difficulty thereof or their number of repetitions.

According to another characteristic of the system of the invention, said communication means comprise a data network, which may be, for example, the Internet, a wide area network (WAN) or a local area network (LAN).

Furthermore, the system may also comprise at least one therapist's computer connected to the central control and processing unit, designed for access to the latter and enable the design and/or selection of an exercise table based on the results of the previous exercises, from the elementary exercises contained in the database.

According to a second aspect of the invention, a task planning method is provided for use in cognitive ability-related treatment, which is essentially characterized in that it comprises the steps of:
- a user making a predefined exercise table,
- automatically entering and collecting the responses in a computer;
- communicating the results of the exercises from the user client computer to a central control and processing unit;
- the central control and processing unit processing the results of the exercise table;
- evaluating the level of correct responses obtained by the user and comparing them with contrast parameters;
- determining the following exercise table selected from a plurality of elementary exercises stored in a database contained and managed by a central control and processing unit; and
- immediately transmitting said following exercise table to the user client computer, or saving the following table to be transmitted in the next connection of the user with the system;
the steps to evaluate and determine being performed by an expert system based on an elementary activity or exercise bank and decision-making criteria implemented in the central control and processing unit.

The expert system may automatically generate the execution environments of the activities for each one of the users, and provide statistical information on the results obtained.

Preferably, the expert system records, in real time, the execution of the personalised plans and the results of each activity or exercise.

According to the invention, the results are recorded and the results are accumulated and presented, together with the user's evaluation, by graphics which show the percentage of success of each exercise table.

The activity or exercise bank manages a plurality of interactive and multimedia activities and elements to parametrize the exercise tables, such as the time of duration thereof, the difficulty thereof or their number of repetitions.

According to another characteristic of the method of the invention, the activities contained in the activity bank are automatically activated by the expert system in accordance with the exercise tables and the progress attained by the user.

The activities are offered to the user by visual and sound media via the computer's peripherals and in a plurality of languages.

Preferably, the exercises are directly performed on computer peripherals in a multimedia environment.

According to a third aspect of the invention, executable instructions are disclosed which, when they are executed by a computer, cause the computer to perform a task planning method for use in cognitive ability-related treatment, which comprises the steps of:
- a user making a predefined exercise table,
- automatically entering and collecting the responses in a computer;
- communicating the results of the exercises from the user client computer to a central control and processing unit;
- the central control and processing unit processing the results of the exercise table;
- an expert system, implemented in the central control and processing unit, evaluating the level of correct responses obtained by the user and comparing them with contrast parameters;
- the expert system determining, based on an elementary activity or exercise bank and decision-making criteria, the following exercise table selected from a plurality of elementary exercises stored in a database contained and managed by a central control and processing unit; and
- immediately transmitting said following exercise table to the user client computer, or saving said following table to be transmitted in the next connection of the user with the system.

In accordance with a fourth aspect, carrier media of the previous instructions executable by computer are disclosed.

Said carrier media may be in the form of storage media or in the form of carrier wave.

According to a fifth aspect, a computer program is disclosed which comprises instructions executable by computer which causes a system for use in cognitive ability-related treatment to perform the aforementioned method.

The present invention enables adapting the field of cognitive psychostimulation to new technologies, turning it into a quick, effective tool which is accessible for professionals, whilst being practical and simple for patients, their carers and family members.

The present invention takes advantage of the potentiality of the data obtained, facilitating the achievement and maintenance of the optimum point of cognitive stress of the patient which encourages increasing and continuous learning without producing frustration due to difficulty or boredom due to simplicity.

The program may have dozens of interactive and multimedia exercises (voice and image) in multiple languages, and grouped by categories, depending on the cognitive functions which have to be stimulated or the deficiencies that one wants to re-establish in each patient. Each of them aims to stimulate a function, such as orientation, attention, language, calculation, gnosis, without the previous list being limitative.

In this way, a very dynamic system is created which enables definition and monitoring by the specialists of stimulation plans for their patients.

The system from the definition of the personalised plans, applies an expert system characterized in that it has decision-making means which automatically generates the activities for each patient based on a bank of previous data.

Each user may have different activities assigned in accordance with the deficiencies in question, and in this way, there is a greater degree of acceptance of the activities or exercises.

### Brief description of the drawings

The attached drawings illustrate, by way of non-limiting example, an embodiment of the task planning system and method for use in the treatment and stimulation of cognitive deficiencies, object of the invention. In said drawings:
- Fig. 1: is a diagram which shows the basic architecture of the system;
- Fig. 2: is a general scheme of the method operation;
- Fig.3: represents a block diagram of the personal environment of the system formed by specialists and therapists;
- Fig. 4: represents a block diagram of the personal environment of the system formed by the patients:
- Figure 5: represents a block diagram of the execution of personalised treatment plans;
- Figure 6: represents a block diagram of the hierarchy and confidentiality of the authorizations;
- Figure 7: represents an access screen to the program for the specialists, with a view of the alerts pending management;
- Figure 8: represents a display and consultation screen for the specialist of one of the personalised treatment plans it has defined;
- Figure 9: represents a display and selection screen for the specialist of the interactive activities available to design personalised plans;
- Figure 10: represents a display screen for the specialist of the results histories of a patient with an exercise;
- Figure 11: represents a display screen for a patient of an interactive exercise.

### Description of preferred embodiments

In particular, the present invention relates to a task planning system for use in the treatment and stimulation of the different cognitive abilities which may have been lost or deteriorated due to congenital or acquired causes, which comprises:
- at least one central control and processing unit or server (B1) which manages:
   o A user database of the system (B4): specialists and patients:
   o A database (B3) with definitions of exercises and methods for the treatment of the various cognitive deficiencies;
   o An expert system (B2) of display and management of personalised treatment plans and automatic adaptation thereof to the development of the users or patients;
- at least one specialist computer (D1) which defines and/or supervises the exercises performed by the patients.
- At least one computer of the patient (A1) or user which performs exercises.
- Communication means (C1) for:
   a) sending the definitions of exercises to be performed by the patients from the specialist's/specialists' computer (D1) to the central server (B1) together with the parameters of development and automatic adaptation thereof to the development shown;
   b) sending the results of the exercises from the patient's computer (A1) to the central server (B1).
   c) sending exercises, proposed by the decision-making means, to the patient's computer (A1);

Hereinafter, the expression "central control and processing unit" and "server" is applied indiscriminately to refer to the same concept, in order to distinguish it from the concept of "client computer."

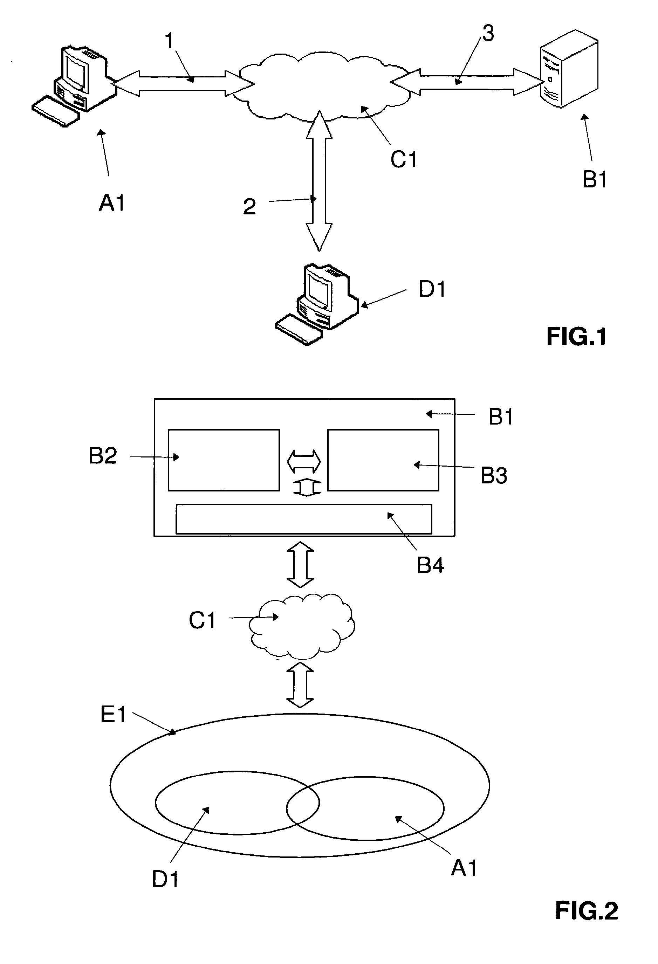

Fig. 1 is a diagram which shows the basic system architecture composed of at least one patient computer (A1), at least one therapist's computer (D1) and a central (B1) server (B1) communicated via a medium (C1).
(A1) represents a computer with access to a data network
(D1) represents a computer with access to a data network.
(C1) represents a data network, which can be a LAN, WAN and a global network, Internet.

(1) represents an abstraction of a communication means from (A1) to (B1) passing through (C1).
(2) represents an abstraction of a communication means from (B1) to (B1) passing through (C1).
(3) represents an abstraction of a communication means from (C1) to (B1).

Fig. 2 is the general scheme of functioning, formed by the central server (B1), within which:
- expert management system (B2), performs:
   o Registration of users and their access codes;
   o Data and client profile management;
   o Definition of personalised treatment plans, for each patient, multilanguage;
   o Maintenance and modification of the personalised plans;
   o Consultation and monitoring, on-line, of the plans, their execution and the results obtained;
   o Internal communication with patients and with other users.
- interactive activity bank (B3), performs:
   o Collection of interactive activities for the acquisition and practice of cognitive abilities: calculation, languages, attention, concentration, memory, gnosis...;
   o Grouping by categories of abilities;
   o Multi-language, culturally adapted activities;
   o Levels of difficulty for each activity;
   o Large collection of multimedia resources to guarantee diversity and that exercises are not repeated;
- personalised treatment plans (B4), contain:
   o Set of activities to perform, and in which order, defined by the expert system (B2) or the specialist, as required;
   o For each activity: initial level of difficulty and the criteria of automatic increase or decrease, display language, number of repetitions;
   o For the personalised plan: definition of the frequency of execution and the daily time limit;

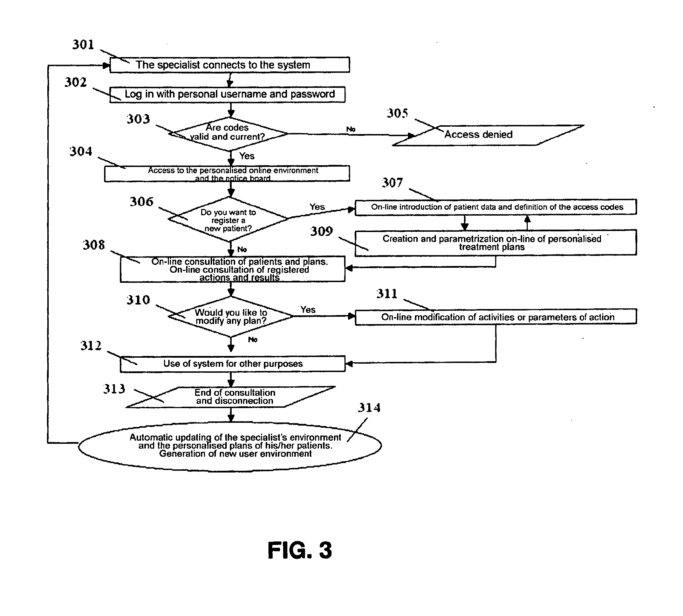

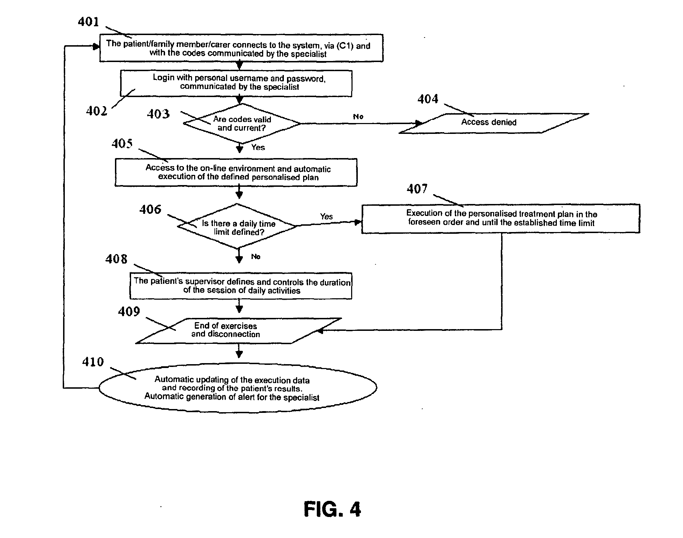

Block (E1) represents the personal environments of the system, in turn composed of:
- specialists and therapists (D1), method of functioning detailed in the block diagram of Fig. 3
- Patients (A1), operating methods detailed in the block diagram of Fig. 4;

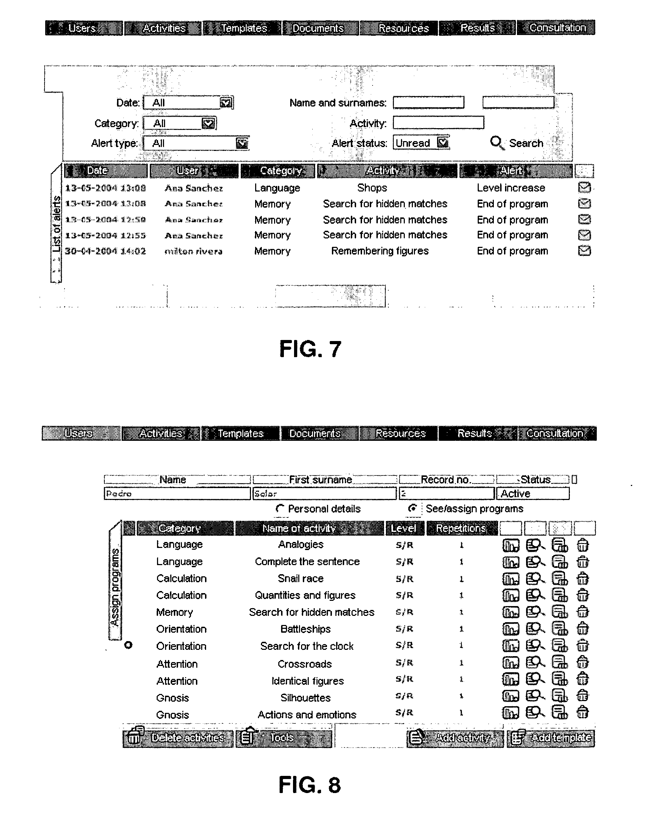

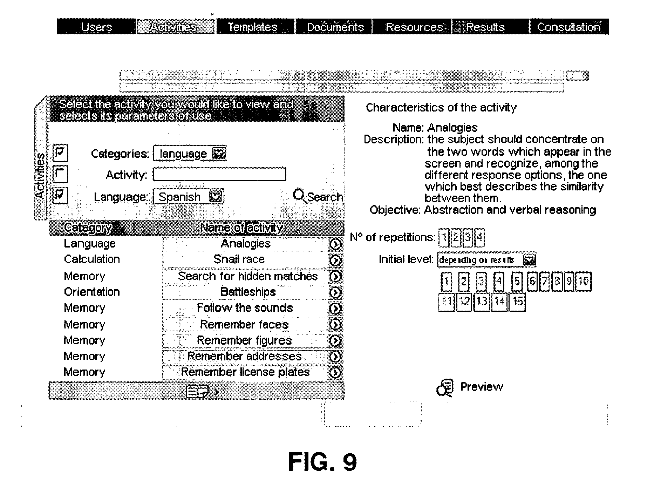

Fig. 3 represents a block diagram of the personal environment of the system formed by specialists and therapists, in step (301) the specialist connects to the system (Fig. 7). Then the username and his/her personal password are logged in (302). If said code is invalid access is denied to the system (305), if it is valid the specialist accesses the on-line environment and the notice board (Fig. 8). He/she can then register more patients (307) with their designed plans (309) and (Fig. 9). He/she can also access consultations of results recorded (Fig. 9).

The personalised environment also includes the possibility that the therapist may modify a plan (310), if relevant, he/she acts in the modification of the parameters of the exercises or activities (311). In the block (313) he/she disconnects and the specialist's environment is updated (314).

Fig. 4 represents a block diagram of the personal environment of the system formed by its patients. The users (401) connect to the system via (C1), they enter their username and password (402), if they are valid the system proceeds, otherwise, access is denied (404). If it proceeds, the user accesses his/her on-line execution environment (405), then the personalised plan starts in the planned order and up to a time limit (Fig. 11). The patient's supervisor defines and controls the duration of the session of the day's activities (408). Once the time limit has been exceeded (406), the exercises end (409) and the execution data is updated and the patient's results are recorded.

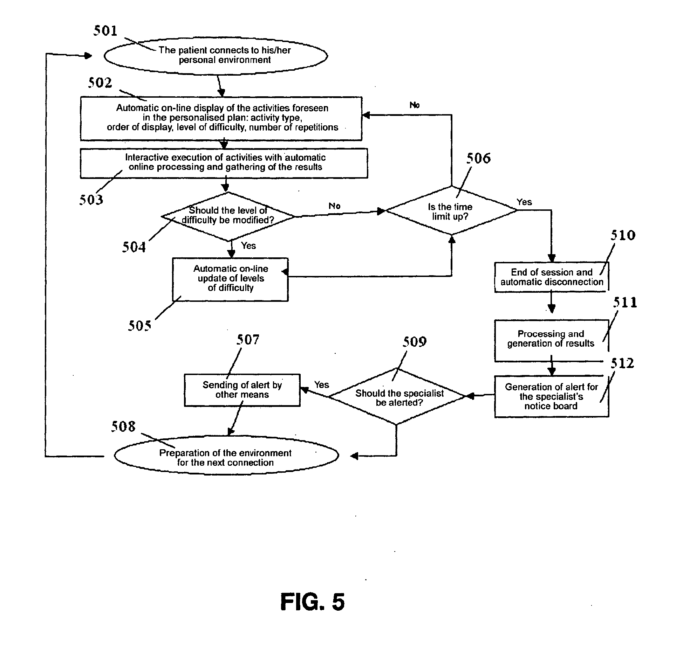

Fig. 5 represents a block diagram of the execution of the personalised treatment plans (B4), which clearly shows the system from the definition of the personalised plans, applies an expert system (B2) characterized by a decision-making means which automatically generates the activities for each patient based on a bank of previous data (B3).

The program is aimed at qualified professionals (301) and is in charge of cognitive psychostimulation of the patients, structured in levels of difficulty, which are automatically activated in accordance with the results (505) and applying a decision-making means, they place at the disposal of the therapist a record of results to monitor the patient's development (512) at the same time the decision-making means feeds on the data obtained to choose the exercises which best benefit the patient with greater precision.

Thanks to the expert system (B2), it offers the possibility of designing personalised treatment plans, taking into consideration the residual abilities of each patient and the characteristics of their environment.

Each user, patient (404) or specialist (303) should log in with a username and a password, previously entered with the user registration method (Figs. 4 and 5).

In this way, the exercises are stored and assigned to each one of the users which are registered in the system, so that when a user logs in the system it automatically knows which exercises the user (405) should have access to.

The user agent interface is a web page based on Macromedia flash language. The transfer protocol between the server (B1) and the user client computer (A1) is *HTTP.*

The program is explicitly designed to be accessed and used via internet, totally on-line, as well as in a local or wide area network (C1). The option of use via Internet facilitates access to the system from any computer with access to the Internet, completely avoiding problems associated with the installation and maintenance of computer programs.

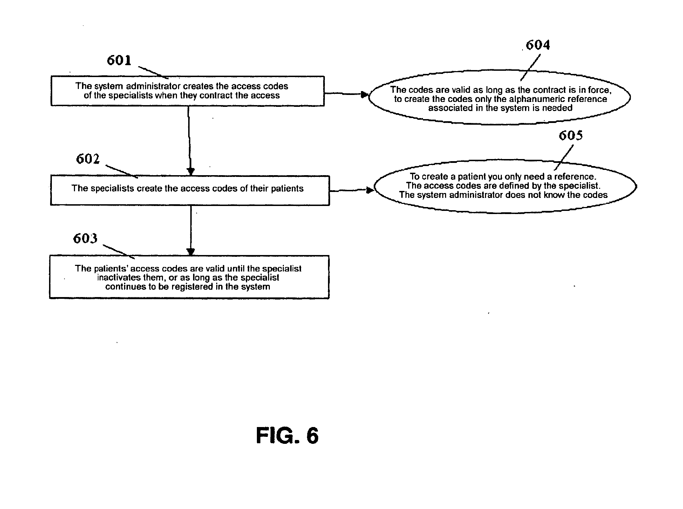

The therapist has a private access (Fig. 3 and Fig. 6) where he/she can access the plans of each one of them, work, modify it and observe the development by recording the results.

To be able to guarantee secure navigation via Internet, reducing the distractions and absent-mindedness of the patient, the activities and exercises are displayed occupying the whole screen, and making all the toolbars disappear both from the browser and the operating system (405).

The patient connects to his/her personal environment (501), the activities foreseen in the personalised plan are displayed automatically and on-line: activity, exercise, level of difficulty... Then these activities or exercises are performed and the results are gathered (503). Fig. 11 shows an example of possible interactive exercise. In this case, the system shows the user an exercise consisting of counting the number of objects that appear onscreen. If the user counts the number correctly, a signal of "exercise passed" is sent to the server.

Depending on the results obtained, the expert system (B2), characterized in that it has a decision-making means, determines if the difficulty should be modified or not. If affirmative, the level (505) is updated and it also checks that the time limit in which to perform the exercises has not been exceeded (506). If negative, the level of difficulty is not modified and it also checks that the time limit in which to perform the exercises has not been exceeded (506).

If the time limit is not exceeded (506) the initial panel is returned to where more activities or exercises are displayed. If the established time is exceeded, an automatic disconnection occurs (510), the results are processed and alerts (511) and (512) are generated.

From the results processing it is decided (509) if the specialist should be alerted. If negative, the environment is prepared for a future connection (508), otherwise it alerts the specialist via communication means (507), for example SMS or by electronic mail (e-mail).

Fig. 6 represents a block diagram of the hierarchy and confidentiality of authorizations, guaranteeing data confidentiality. In step (601) the system administrator creates the access codes of the specialist when they contract the access, said codes are valid as long as there is a contract in force (604). Afterwards, it is the specialists who create their patients' access codes (602), to create a patient user all that is needed is a reference and an access code, so that the system administrator does not know the patients' codes (605). The patient user codes remain active until blocked by the specialist (603).

Fig. 7 represents an access screen to the program for the specialist, with a view of the alerts pending management. In this case an ordered alerts list is obtained, it is also possible to carry our searches based on the patient's name, activity, if he/she has read the message or not, by dates, categories and alert type.

Fig. 8 represents a display and consultations screen for the specialist of one of the personalised treatment plans that he/she has defined. In this place he/she can assign a personalised plan to each user, it is possible to modify activities (add activity, add template, delete activity, tools), the number of repetitions, the level and the category.

Fig. 9 represents a display and selection screen for the specialist of the interactive activities available to design personalised plans. In this case the characteristics and data of the activity can be specified, selecting the category, the language (which may be different for each individual activity), the number of repetitions thereof and the level of difficulty. Once the specification has concluded it is possible to perform a pre-view.

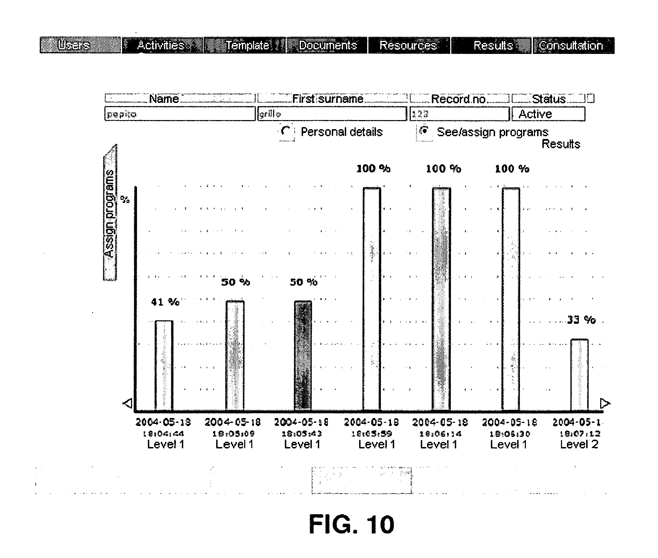

Fig. 10 represents a display screen for the specialist of the history of a patient with an exercise. The graphic shown onscreen indicates the statistics of each activity performed, as well as also the personal data of the user who has completed it. Each bar signifies the percentage of success of each exercise or activity, and below (x-axis) it shows the date, time and performance level.

The system and method of the present specification is advantageous, since it makes use of all the potentiality of the data obtained, facilitating the achievement and maintenance of the optimum point of cognitive stress of the patient which encourages increasing and continuous learning without producing frustration due to difficulty or boredom due to simplicity.

To facilitate the interpretation of Figs. 3, 4, 5, and 6 this table is included which shows the correspondence between the numerical label and the corresponding action.

| Number | Action |
|---|---|
| 301 | The specialist connects to the system |
| 302 | Log in with personal username and password |
| 303 | Are codes valid and current? |
| 304 | Access to the personalised online environment and the notice board |
| 305 | Access denied |
| 306 | Do you want to register a new patient? |
| 307 | On-line introduction of patient data and definition of the access codes |
| 308 | On-line consultation of patients and plans. On-line consultation of registered actions and results |
| 309 | Creation and parametrization on-line of personalised treatment plans |
| 310 | Would you like to modify any plan? |
| 311 | On-line modification of activities or parameters of action |
| 312 | Use of system for other purposes |
| 313 | End of consultation and disconnection |
| 314 | Automatic updating of the specialist's environment and the personalised plans of his/her patients. Generation of new user environment. |
| 401 | The patient/family member/carer connects to the system, via (C1) and with the codes communicated by the specialist |
| 402 | Login with personal username and password, communicated by the specialist |
| 403 | Are codes valid and current? |
| 404 | Access denied |
| 405 | Access to the on-line environment and automatic execution of the defined personalised plan |
| 406 | Is there a daily time limit defined? |
| 407 | Execution of the personalised treatment plan in the foreseen order and until the established time limit |
| 408 | The patient's supervisor defines and controls the duration of the session of daily activities |
| 409 | End of exercises and disconnection |
| 410 | Automatic updating of the execution data and recording of the patient's results. Automatic generation of alert for the specialist. |
| 501 | The patient connects to his/her personal environment |
| 502 | Automatic on-line display of the activities foreseen in the personalised plan: activity type, order of display, level of difficulty, number of repetitions |
| 503 | Interactive execution of activities with automatic online processing and gathering of the results |
| 504 | Should the level of difficulty be modified? |
| 505 | Automatic on-line update of levels of difficulty |
| 506 | Is the time limit up? |
| 507 | Sending of alert by other means |
| 508 | Preparation of the environment for the next connection |
| 509 | Should the specialist be alerted? |
| 510 | End of session and automatic disconnection |
| 511 | Processing and generation of results |
| 512 | Generation of alert for the specialist's notice board |
| 601 | The system administrator creates the access codes of the specialists when they contract the access |
| 602 | The specialists create the access codes of their patients |
| 603 | The patients' access codes are valid until the specialist inactivates them, or as long as the specialist continues to be registered in the system |
| 604 | The codes are valid as long as the contract is in force, to create the codes only the alphanumeric reference associated in the system is needed. |
| 605 | To create a patient you only need a reference. The access codes are defined by the specialist. The system administrator does not know the codes. |

Having sufficiently described the nature of the present invention, as well as the form of putting it into practice, it is stated that provided that its fundamental principle is not altered, changed or modified, it is subject to detail changes.

## Claims

1. System for task planning for use in cognitive ability-related treatment which comprises at least one central control and processing unit (B1) which manages a database of users and definitions of exercises and methods for cognitive ability-related treatment, **characterized in that** it comprises a network of computers connected to the central control and processing unit (B1) with:
- at least one user client computer (A1) performing exercises; and
- communication means between the central control and processing unit (B1) and the user client computer, designed to send definitions of exercises from the central control and processing unit (B1) to the user client computer (A1) that are proposed by decision-making means and to send the results of the exercises from the user client computer (A1) to the central control and processing unit (B1), said decision-making means comprising an expert system (B2) implemented in the central control and processing unit (B1), designed to determine automatically exercises to be performed by the user in a personalised manner, such that they are adapted to his/her development and to modify the content of the memory of the central control and processing unit (B1).

2. System according to claim 1, **characterized in that** said database contains definitions of elementary exercises, from which the expert system (B2) determines an exercise table.

3. System according to claim 2, **characterized in that** said exercise tables are combinations of elementary exercises and criteria of parametrization, such as the time of duration thereof, the difficulty thereof or their number of repetitions.

4. System according to claim 1, **characterized in that** said communication means comprise a data network.

5. System according to claim 4, **characterized in that** said data network is Internet.

6. System according to claim 4, **characterized in that** said data network is a wide area network (WAN).

7. System according to claim 4, **characterized in that** said data network is a local area network (LAN).

8. System according to any of the preceding claims, **characterized in that** it further comprises at least one therapist's computer (D1) connected to the central control and processing unit (B1) designed for access to the latter and enable the design and/or selection of an exercise table based on the results of the previous exercises, from the elementary exercises contained in the database.

9. Task planning method for use in cognitive ability-related treatment, wherein a user makes a predefined exercise table, and enters the results in a computer, in a system according to claims 1 to 8, **characterized in that** it comprises the steps of:
- automatically entering and collecting the responses in a computer;
- communicating the results of the exercises from the user client computer (A1) to a central control and processing unit (B1);
- the central control and processing unit (B1) processing the results of the exercise table;
- evaluating the level of correct responses obtained by the user and comparing them with contrast parameters;
- determining the following exercise table selected from a plurality of elementary exercises stored in a database contained and managed by a central control and processing unit (B1); and
- transmitting said following exercise table to the user client computer (A1), or saving the following table to be transmitted in the next connection of the user with the system;
the steps to evaluate and determine being performed by an expert system (B2) based on a bank of elementary activities or exercises and decision-making criteria (B3) implemented in the central control and processing unit (B1).

10. Method, according to claim 9, **characterized in that** the expert system (B2) automatically generates the execution environments of the activities for each one of the users.

11. Method, according to claim 10, **characterized in that** the expert system (B2) provides statistical information on the results obtained.

12. Method, according to claim 11, **characterized in that** the expert system (B2) records, in real time, the execution of the personalised plans and the results of each activity or exercise.

13. Method, according to claim 12, **characterized in that** the results are recorded and accumulated and they are displayed, together with the user's development, by graphics which show the percentage of success of each exercise table.

14. Method, according to claim 9, **characterized in that** the activity or exercise bank (B3) manages a plurality of interactive and multimedia services and elements to parametrize the exercise tables, such as the time of duration thereof, the difficulty thereof or their number of repetitions.

15. Method, according to claim 14, **characterized in that** the activities contained in the activity bank (B3) are automatically activated by the expert system (B2) depending on the exercise tables and the progress attained by the user.

16. Method, according to claim 15, **characterized in that** the activities are offered to the user by visual and sound media via the computer's peripherals.

17. Method, according to claim 16, **characterized in that** the activities are offered to the user in a plurality of languages.

18. Method, according to claim 15, **characterized in that** the exercises are directly performed on peripherals of the computer (A1) in a multi-language environment.

19. Instructions executable by computer which, when they are executed by a computer, make the computer perform a task planning method according to claims 1 to 8.

20. Carrier media of the instructions executable by computer of claim 19.

21. Carrier media according to claim 20, which is presented in the form of storage media.

22. Carrier media according to claim 20, which is presented in the form of a carrier wave.

23. Computer program which comprises instructions executable by computer which makes a system for cognitive ability-related treatment perform the method according to any of claims 9 to 18.
